# EUROPEAN PATENT APPLICATION

(11) **EP 4 368 176 A1**
(43) Date of publication of application: **15.05.2024**
(21) Application number: 22837758.6
(22) Date of filing: 08.07.2022
(51) Int. Cl.: A61K 31/047, A61K 31/7088, A61K 31/712, A61K 31/7125, A61K 47/26, A61P 13/12, A61P 39/02, C12N 15/113

(54) **NEPHROTOXICITY REDUCING AGENT**

(30) Priority: 08.07.2021 JP 2021113495
(71) Applicant: Nippon Shinyaku Co., Ltd., Kyoto-shi Kyoto 601-8550 (JP); National University Corporation Kobe University, Kobe-shi, Hyogo 657-8501 (JP)
(72) Inventor: SONOKE Satoru, Kyoto-shi, Kyoto 601-8550 (JP); FUJIWARA Kae, Kyoto-shi, Kyoto 601-8550 (JP); SATOU Youhei, Kyoto-shi, Kyoto 601-8550 (JP); WAKAYAMA Tatsushi, Tsukuba-shi, Ibaraki 305-0003 (JP); MASUDA Hirofumi, Tsukuba-shi, Ibaraki 305-0003 (JP); SEKI Ryosuke, Tsukuba-shi, Ibaraki 305-0003 (JP); MATSUBARA Takuma, Tsukuba-shi, Ibaraki 305-0003 (JP); NUMAKURA Yuki, Kyoto-shi, Kyoto 601-8550 (JP); OKAMOTO Kentaro, Kyoto-shi, Kyoto 601-8550 (JP); TODA Tatsushi, Kobe-shi, Hyogo 657-8501 (JP); IKEDA Mariko, Kobe-shi, Hyogo 657-8501 (JP); KOBAYASHI Kazuhiro, Kobe-shi, Hyogo 657-8501 (JP)
(74) Representative: J A Kemp LLP
(86) International application number: PCT/JP2022/027103
(87) International publication number: WO 2023/282344

(57) **Abstract**

In one embodiment, the object of the present invention is to provide a nephrotoxicity reducing agent for a pharmaceutical composition comprising an antisense oligomer, and a method for reducing nephrotoxicity of the pharmaceutical composition. In one embodiment, the present invention relates to a nephrotoxicity reducing agent for a pharmaceutical composition comprising an antisense oligomer, wherein the nephrotoxicity reducing agent comprises a sugar alcohol and is used in an amount such that the concentration of the sugar alcohol in the pharmaceutical composition is 1 mg/mL to 400 mg/mL.

## Description

### Technical Field

The present invention relates to a nephrotoxicity reducing agent for a pharmaceutical composition comprising an antisense oligomer, a method for reducing nephrotoxicity of the pharmaceutical composition, a pharmaceutical composition comprising an antisense oligomer with reduced nephrotoxicity, and the like.

### Background Art

Antisense oligomers are nucleic acids that sequence-specifically hybridize to target mRNAs and pre-mRNAs. The antisense oligomers exert their actions by degradation of mRNAs and pre-mRNAs, exon-skipping, exon-inclusion, translation inhibition and the like, and thus have been used as therapeutic agents for some diseases. For example, Japanese Patent Laid-Open No. 2015-91229 (Patent Literature 1) discloses an antisense nucleic acid which can treat Fukuyama muscular dystrophy by normalizing aberrant splicing of a fukutin gene having an insertion mutation of SVA retrotransposon.

However, with respect to the antisense oligomers, it have been known that, for example, morpholino oligomers are accumulated in the kidney (Non Patent Literature 1, Figure 2), administration of morpholino oligomers causes nephrotoxicity to be expressed (Non Patent Literature 2, Figure 3, Table 5), and the administration of morpholino oligomers induces basophilic substances in the kidney tubule (Non Patent Literature 3, Figure 1). A method for avoiding the appearance of the nephrotoxicity and the basophilic substances in the renal tubule has not been established. On the other hand, it has been known the following: for oligonucleotides that have greater toxicity in the aggregate form than in monomeric form, heating with use of chemical species such as mannitol that disrupt aggregates can suppress multimerization in formulations of therapeutic oligonucleotides (Patent Literature 2); and saccharides and sugar alcohols in liquid formulations suppress aggregation of oligonucleotides (Patent Literature 3).

### Citation List

### Patent Literature

Patent Literature 1: Japanese Patent Laid-Open No. 2015-91229
Patent Literature 2: International Publication No. 2002/036767
Patent Literature 3: International Publication No. 2010/009038

### Non Patent Literature

Non Patent Literature 1: Heemskerk HA, de Winter CL, de Kimpe SJ, van Kuik-Romeijn P, Heuvelmans N, Platenburg GJ, van Ommen GJ, van Deutekom JC, Aartsma-Rus A. In vivo comparison of 2'-O-methyl phosphorothioate and morpholino antisense oligonucleotides for Duchenne muscular dystrophy exon skipping. J Gene Med. 2009 Mar; 11(3):257-66. doi: 10.1002/jgm.1288.
Non Patent Literature 2: Sazani P, Ness KP, Weller DL, Poage D, Nelson K, Shrewsbury AS. Chemical and mechanistic toxicology evaluation of exon skipping phosphorodiamidate morpholino oligomers in mdx mice. Int J Toxicol. 2011 May; 30(3):322-33. doi: 10.1177/1091581811403504. Epub 2011 May 3.
Non Patent Literature 3: Carver MP, Charleston JS, Shanks C, Zhang J, Mense M, Sharma AK, Kaur H, Sazani P. Toxicological Characterization of Exon Skipping Phosphorodiamidate Morpholino Oligomers (PMOs) in Non-human Primates. J Neuromuscul Dis. 2016 Aug 30; 3(3):381-393. doi: 10.3233/JND-160157.

### Summary of Invention

### Technical Problem

It has conventionally been known that pharmaceutical compositions comprising antisense oligomers have a nephrotoxicity problem. In such a circumstance, it is desirable to provide an improved pharmaceutical composition comprising antisense oligomers.

### Solution to Problem

That is, the present invention provides a nephrotoxicity reducing agent for a pharmaceutical composition comprising an antisense oligomer, a method for reducing nephrotoxicity of the pharmaceutical composition, a pharmaceutical composition comprising an antisense oligomer with reduced nephrotoxicity, and the like as follows.
(1) A nephrotoxicity reducing agent for a pharmaceutical composition comprising an antisense oligomer, wherein the nephrotoxicity reducing agent comprises a sugar alcohol and is used in an amount such that the concentration of the sugar alcohol in the pharmaceutical composition is 1 mg/mL to 400 mg/mL.
(2) The nephrotoxicity reducing agent according to (1), wherein the nephrotoxicity reducing agent is used in an amount such that the concentration of the sugar alcohol in the pharmaceutical composition is 2.5 mg/mL to 200 mg/mL.
(3) The nephrotoxicity reducing agent according to (1) or (2), wherein a concentration of the antisense oligomer in the pharmaceutical composition is 0.5 mg/mL to 200 mg/mL.
(4-1) A nephrotoxicity reducing agent for a pharmaceutical composition comprising an antisense oligomer, wherein the nephrotoxicity reducing agent comprises a sugar alcohol and is used in an amount such that the weight ratio of the sugar alcohol is 0.05 to 30 per 1 of the antisense oligomer.
(4-2) A nephrotoxicity reducing agent for a pharmaceutical composition comprising an antisense oligomer, wherein the nephrotoxicity reducing agent comprises a sugar alcohol and is used in an amount such that the weight ratio of the sugar alcohol is 0.05 to 90 per 1 of the antisense oligomer.
(4-3) The nephrotoxicity reducing agent according to (4-1) or (4-2), wherein the pharmaceutical composition and the nephrotoxicity reducing agent are administered separately.
(5) The nephrotoxicity reducing agent according to any of (4-1) to (4-3), wherein the nephrotoxicity reducing agent is used in an amount such that the weight ratio of the sugar alcohol is 0.1 to 13.3 per 1 of the antisense oligomer.
(6) The nephrotoxicity reducing agent according to any of (1) to (5), wherein the sugar alcohol is selected from the group consisting of mannitol, sorbitol, and a combination thereof.
(7) The nephrotoxicity reducing agent according to any of (1) to (6), wherein the antisense oligomer is a morpholino oligomer.
(8) The nephrotoxicity reducing agent according to any of (1) to (7), wherein the antisense oligomer is a phosphorodiamidate morpholino oligomer.
(9) The nephrotoxicity reducing agent according to any of (1) to (8), wherein the 5'-end of the antisense oligomer is any group represented by the following chemical formulae (1) and (2): 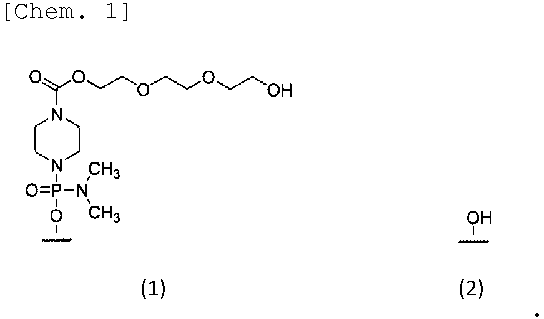
(10) The nephrotoxicity reducing agent according to any of (1) to (9), wherein the antisense oligomer comprises four consecutive purine bases in its base sequence.
(11) The nephrotoxicity reducing agent according to (10), wherein at least two of the four consecutive purine bases are guanine.
(12) The nephrotoxicity reducing agent according to any of (1) to (11), wherein the antisense oligomer comprises a base sequence selected from the group consisting of SEQ ID NOs: 1 to 10.
(13) The nephrotoxicity reducing agent according to any of (1) to (12), wherein the antisense oligomer targets a sequence ranging from positions 115937 to 115981 of a base sequence set forth as SEQ ID NO: 11.
(14) The nephrotoxicity reducing agent according to any of (1) to (12), wherein the antisense oligomer does not target a sequence ranging from positions 115937 to 115981 of a base sequence set forth as SEQ ID NO: 11.
(15-1) A method for reducing nephrotoxicity for a pharmaceutical composition comprising an antisense oligomer, comprising adding a sugar alcohol in an amount such that the concentration of the sugar alcohol in the pharmaceutical composition is 1 mg/mL to 400 mg/mL.
(15-2) The method according to (15-1), wherein the sugar alcohol is added in an amount such that the concentration of the sugar alcohol in the pharmaceutical composition is 2.5 mg/mL to 200 mg/mL.
(15-3) The method according to (15-1) or (15-2), wherein the concentration of the antisense oligomer in the pharmaceutical composition is 0.5 mg/mL to 200 mg/mL.
(16-1) A method for reducing nephrotoxicity of an antisense oligomer in a subject to whom the antisense oligomer has been administered, comprising administering a sugar alcohol to the subject, wherein the sugar alcohol is administered in an amount such that the weight ratio of the sugar alcohol is 0.05 to 30 per 1 of the antisense oligomer.
(16-2) A method for reducing nephrotoxicity of an antisense oligomer in a subject to whom the antisense oligomer has been administered, comprising administering a sugar alcohol to the subject, wherein the sugar alcohol is administered in an amount such that the weight ratio of the sugar alcohol is 0.05 to 90 per 1 of the antisense oligomer.
(16-3) The method according to (16-1) or (16-2), wherein the antisense oligomer and the sugar alcohol are administered separately.
(16-4) The method according to any of (16-1) to (16-3), wherein the sugar alcohol is used in an amount such that the weight ratio of the sugar alcohol is 0.1 to 13.3 per 1 of the antisense oligomer.
(17-1) A pharmaceutical composition comprising an antisense oligomer with reduced nephrotoxicity, wherein the pharmaceutical composition comprises a nephrotoxicity reducing agent comprising a sugar alcohol at a concentration of 1 mg/mL to 400 mg/mL.
(17-2) The pharmaceutical composition according to (17-1), wherein the pharmaceutical composition comprises the nephrotoxicity reducing agent comprising a sugar alcohol at a concentration of 2.5 mg/mL to 200 mg/mL.
(17-3) The pharmaceutical composition according to (17-1) or (17-2), wherein the concentration of the antisense oligomer in the pharmaceutical composition is 0.5 mg/mL to 200 mg/mL.
(18) The method or pharmaceutical composition according to any of (15-1) to (17-3), wherein the sugar alcohol is selected from the group consisting of mannitol, sorbitol, and a combination thereof.
(19) The method or pharmaceutical composition according to any of (15-1) to (18), wherein the antisense oligomer is a morpholino oligomer.
(20) The method or pharmaceutical composition according to any of (15-1) to (19), wherein the antisense oligomer is a phosphorodiamidate morpholino oligomer.
(21) The method or pharmaceutical composition according to any of (15-1) to (20), wherein the 5'-end of the antisense oligomer is any group represented by the following chemical formulae (1) and (2): 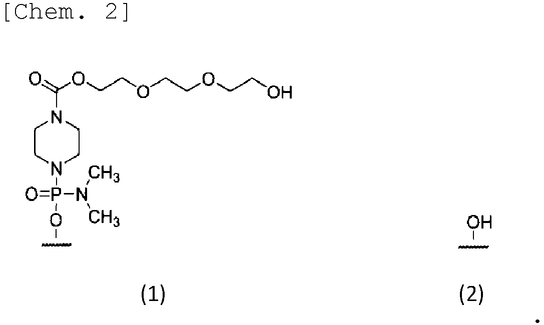
(22) The method or pharmaceutical composition according to any of (15-1) to (21), wherein the antisense oligomer comprises four consecutive purine bases in its base sequence.
(23) The method or pharmaceutical composition according to (22), wherein at least two of the four consecutive purine bases are guanine.
(24) The method or pharmaceutical composition according to any of (15-1) to (23), wherein the antisense oligomer comprises a base sequence selected from the group consisting of SEQ ID NOs: 1 to 10.
(25) The nephrotoxicity reducing agent according to any of (15-1) to (24), wherein the antisense oligomer targets a sequence ranging from positions 115937 to 115981 of a base sequence set forth as SEQ ID NO: 11.
(26) The nephrotoxicity reducing agent according to any of (15-1) to (24), wherein the antisense oligomer does not target a sequence ranging from positions 115937 to 115981 of a base sequence set forth as SEQ ID NO: 11.

### Advantageous Effect of Invention

The present invention provides a nephrotoxicity reducing agent for a pharmaceutical composition comprising an antisense oligomer, and a method for reducing nephrotoxicity of the pharmaceutical composition.

### Brief Description of Drawings

[Figure 1] Figure 1 shows absorbance measurement values at 620 nm under the conditions shown in Table 11.
[Figure 2] Figure 2 shows absorbance measurement values at 620 nm under the conditions shown in Table 12.
[Figure 3] Figure 3 shows absorbance measurement values at 620 nm under the conditions shown in Table 13.
[Figure 4] Figure 4 shows absorbance measurement values at 620 nm under the conditions shown in Table 14.
[Figure 5] Figure 5 shows absorbance measurement values at 620 nm under the conditions shown in Table 15.
[Figure 6] Figure 6 shows absorbance measurement values at 620 nm under the conditions shown in Table 16.

### Description of Embodiments

In one embodiment, the present invention relates to a nephrotoxicity reducing agent for a pharmaceutical composition comprising an antisense oligomer. The term "nephrotoxicity reducing agent" as used herein means an agent for reducing nephrotoxicity or an event that causes the nephrotoxicity (e.g., accumulation of basophilic substances) that a pharmaceutical composition comprising an antisense oligomer may have. Distribution and accumulation of administered antisense oligomers at a high concentration in the kidney during the excretion process may cause basophilic substances to be expressed in the kidney. The term nephrotoxicity means occurrence of tissue damage or a decrease in kidney function due to higher accumulation of the antisense oligomers in the kidney. The tissue damage in the kidney is known to cause, for example, expansion and necrosis of renal tubule, and the decrease in kidney function is widely known to cause an increase in blood urea nitrogen (BUN) values and blood creatinine (Cre) values, for example. By reducing the nephrotoxicity of the antisense oligomers, a pharmaceutical composition comprising a high dose of antisense oligomers, and a method for treating diseases using the pharmaceutical composition can be provided.

The presence or absence of nephrotoxicity reducing effects can be determined, for example, by measuring the blood urea nitrogen (BUN) values and blood creatinine (Cre) values with, for example, a urease-GIDH method and an enzymatic method, respectively, as described in Examples. For example, it can be determined to be effective in reducing nephrotoxicity when the BUN values and/or Cre values are reduced, for example, by 5% or more, 100 or more, 20% or more, 30% or more, 400 or more, or 50% or more in the case of administering the nephrotoxicity reducing agent, compared to a case of administering no nephrotoxicity reducing agent.

In one embodiment, the nephrotoxicity reducing agent of the present invention may consist of or comprise a sugar alcohol. Examples of the sugar alcohol include, but not limited to, mannitol, sorbitol, glycerol, xylitol, arabitol, erythritol, galactitol, fucitol, iditol, inositol, volemitol, and lactitol. In one embodiment, the sugar alcohol is selected from the group consisting of mannitol, sorbitol, glycerol, and a combination thereof, and preferably, selected from the group consisting of mannitol, sorbitol, and a combination thereof.

When the nephrotoxicity reducing agent of the present invention comprises an ingredient other than the sugar alcohol, it can be formulated by appropriately blending a pharmaceutically acceptable carrier or additive (and optionally the nephrotoxicity reducing agent of the present invention) to the sugar alcohol. Specifically, it can be formulated into oral agents such as tablets, coated tablets, pills, powders, granules, capsules, liquids, suspensions, and emulsions; and parenteral agents such as injectables, infusions, suppositories, ointments and patches. The parenteral agents are preferable. The injectables may be lyophilized preparations. The blending proportion of the carrier or additive can be appropriately set according to the range usually employed in the pharmaceutical field. Examples of the carriers or additives to be blended include, but not particularly limited, various carriers such as water, physiological saline, other aqueous solvents, and aqueous or oily bases, and various additives such as excipients, binders, pH adjusters, disintegrants, absorption promoters, lubricants, colorants, corrigents, and flavors.

The additives miscible with tablets, capsules or the like are used that include, for example, binders such as gelatin, corn starch, tragacanth, or gum arabic, excipients such as crystalline cellulose, swelling agents such as corn starch, gelatin, or alginic acid, lubricants such as magnesium stearate, sweetening agents such as sucrose, lactose, or saccharin, flavoring agents such as peppermint, Gaultheria adenothrix oil, or cherries. When the formulated dosage unit form is a capsule, a liquid carrier such as oil/fat can be further comprised in the materials of the above types. A sterile composition for injection can be prepared according to the normal pharmaceutical practice (e.g., dissolution or suspension of active ingredients into solvents such as water for injection, or natural vegetable oil). As the aqueous solutions for injection, isotonic solutions (e.g., sodium chloride) comprising physiological saline, glucose, or other aid agents are used and can be combined with an adequate solubilizer such as alcohol (e.g., ethanol), polyalcohol (e.g., propylene glycol or polyethylene glycol), and a nonionic surfactant (e.g., polysorbate 80 (TM) or HCO-50). As the oily solutions, for example, sesame oil or soybean oil is used and can be combined with a solubilizer, such as benzyl benzoate or benzyl alcohol. In addition, buffers (e.g., phosphate buffer solution or sodium acetate buffer solution), soothing agents (e.g., benzalkonium chloride or procaine hydrochloride), stabilizers (e.g., human serum albumin or polyethylene glycol), preservatives (e.g., benzyl alcohol or phenol), or antioxidants may be blended therewith. Further, it can be lyophilized preparations.

As used herein, the antisense oligomer may be any of an oligonucleotide, a morpholino oligomer, or a peptide nucleic acid (PNA) oligomer (hereinafter, also referred to as "antisense oligonucleotide described herein", "antisense morpholino oligomer described herein", "antisense peptide nucleic acid oligomer described herein", respectively).

The antisense oligonucleotide is an antisense oligomer whose constituent unit is a nucleotide, and the nucleotide may be any of a ribonucleotide, a deoxyribonucleotide, or a modified nucleotide.

The modified nucleotide refers to those in which all or part of nucleobase, sugar moiety and phosphate-binding moiety constituting ribonucleotides or deoxyribonucleotides are modified.

Examples of the nucleobases can include adenine, guanine, hypoxanthine, cytosine, thymine, uracil, or modified bases thereof. Examples of the modified bases include, but not limited to, pseudouracil, 3-methyluracil, dihydrouracil, 5-alkynecytosine (e.g., 5-methylcytosine), 5-alkyluracil (e.g., 5-ethyluracil), 5-halouracil (e.g., 5-bromouracil), 6-azapyrimidine, 6-alkylpyrimidine (e.g., 6-methyluracil), 2-thiouracil, 4-thiouracil, 4-acetylcytosine, 5-(carboxyhydroxymethyl)uracil, 5-carboxymethylaminomethyl-2-thiouracil, 5-carboxymethylaminomethyl uracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2,6-diaminopurine, 2-aminopurine, isoguanine, indole, imidazole, and xanthine.

Examples of the modification of the sugar moiety can include modification at the 2' position of ribose, and modification of other parts of the sugar. Examples of the modification at the 2' position of ribose can include modification wherein -OH group at the 2' position of ribose is substituted with -OR, -R, -R'OR, -SH, -SR, -NH₂, -NHR, -NR₂, -N₃, -CN, -F, -Cl, -Br, and -I. Here, R represents alkyl or aryl. R' represents alkylene.

Examples of the modification of other parts of the sugar include, but not limited to, those in which O at the 4' position of ribose or deoxyribose is substituted with S, and the 2' and 4' positions of the sugar are cross-linked, for example, a locked nucleic acid (LNA) or 2'-0,4'-C-ethylene-bridged nucleic acid (ENA).

Examples of the modification of phosphate-binding moiety can include modifications wherein the phosphodiester bond is substituted with a phosphorothioate bond, a phosphorodithioate bond, an alkylphosphonate bond, a phosphoramidate bond, and a boranophosphate bond (see, e.g., Enya et al: Bioorganic & Medicinal Chemistry, 2008, 18, 9154-9160) (see, e.g., Japanese Re-Publication of International Patent Application Nos. 2006/129594 and 2006/038608).

As used herein, the alkyl is preferably a linear or branched alkyl having 1 to 6 carbon atoms. Specific examples thereof include methyl, ethyl, n-propyl, isopropyl, n-butyl, isobutyl, sec-butyl, tert-butyl, n-pentyl, isopentyl, neopentyl, tert-pentyl, n-hexyl, and isohexyl. The alkyl may be substituted, examples of the substituent therefor can include halogen, alkoxy, cyano, and nitro, and 1 to 3 positions may be substituted with these substituents.

As used herein, the cycloalkyl is preferably those having 3 to 12 carbon atoms. Specific examples thereof include cyclopropyl, cyclobutyl, cyclopentyl, cyclohexyl, cycloheptyl, cyclooctyl, cyclodecyl, and cyclododecyl.

As used herein, examples of the halogen can include fluorine, chlorine, bromine, and iodine.

As used herein, examples of the alkoxy include a linear or branched alkoxy having 1 to 6 carbon atoms, and for example, methoxy, ethoxy, n-propoxy, isopropoxy, n-butoxy, isobutoxy, sec-butoxy, tert-butoxy, n-pentyloxy, isopentyloxy, n-hexyloxy, and isohexyloxy. Alkoxy having 1 to 3 carbon atoms is preferable among others.

As used herein, the aryl is preferably those having 6 to 10 carbon atoms. Specific examples thereof can include phenyl, α-naphthyl, and β-naphthyl. Phenyl is preferable among others. The aryl may be substituted, examples of the substituent therefor can include alkyl, halogen, alkoxy, cyano, and nitro, and the aryl may be substituted with 1 to 3 of these substituents.

As used herein, the alkylene is preferably a linear or branched alkylene having 1 to 6 carbon atoms. Specific examples thereof can include methylene, ethylene, trimethylene, tetramethylene, pentamethylene, hexamethylene, 2-(ethyl)trimethylene, and 1-(methyl)tetramethylene.

As used herein, examples of acyl can include a linear or branched alkanoyl or aroyl. Examples of the alkanoyl can include formyl, acetyl, 2-methylacetyl, 2,2-dimethylacetyl, propionyl, butyryl, isobutyryl, pentanoyl, 2,2-dimethylpropionyl, and hexanoyl. Examples of the aroyl can include benzoyl, toluoyl, and naphthoyl. The aroyl may be substituted at a substitutable position, or may be substituted with alkyl.

The antisense oligonucleotide described herein can be easily synthesized with various automated synthesizers (e.g., AKTA oligopilot plus 10 / 100 (GE Healthcare)). Alternatively, it can be made by entrusting any third party institution (e.g., Promega Corporation or Takara Bio Inc.).

The antisense morpholino oligomer described herein is an antisense oligomer whose constituent unit is a group represented by the following general formula: 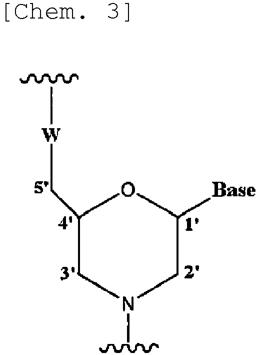 wherein, Base represents a nucleobase; and
W represents a group represented by any of the following formulae: 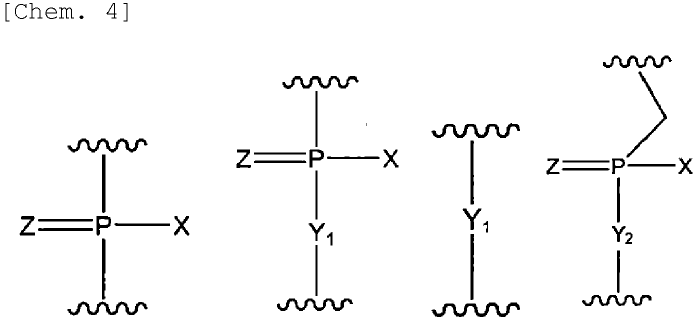 wherein, X represents -CH₂R¹, -O-CH₂R¹, -S-CH₂R¹, -NR²R³, or F;
R¹ represents H, or alkyl;
R² and R³ are the same or different, and represent H, alkyl, cycloalkyl, or aryl;
Y₁ represents O, S, CH₂, or NR¹;
Y₂ represents O, S, or NR¹; and
Z represents O or S.

As used herein, the morpholino oligomer is preferably an oligomer whose constituent unit is a group represented by the following formula (phosphorodiamidate morpholino oligomer, hereinafter referred to as "PMO"): 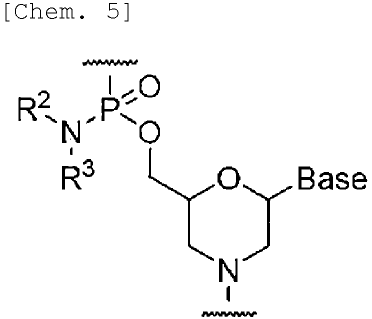 wherein, Base, R² and R³ are as defined above.

The morpholino oligomer can be produced according to the methods described in International Publication Nos. 1991/009033, or 2009/064471, for example. Particularly, PMO can be produced according to the methods described in International Publication Nos. 2009/064471, or 2013/100190.

The antisense peptide nucleic acid oligomer described herein is an antisense oligomer whose constituent unit is a group represented by the following general formula: 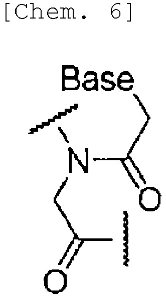 wherein, Base is as defined above.

The peptide nucleic acid oligomer can be produced, for example, according to the following references:
1) P. E. Nielsen, M. Egholm, R. H. Berg, O. Buchardt, Science, 254, 1497 (1991);
2) M. Egholm, O. Buchardt, P. E. Nielsen, R. H. Berg, JACS, 114, 1895 (1992);
3) K. L. Dueholm, M. Egholm, C. Behrens, L. Christensen, H. F. Hansen, T. Vulpius, K. H. Petersen, R. H. Berg, P. E. Nielsen, O. Buchardt, J. Org. Chem., 59, 5767 (1994);
4) L. Christensen, R. Fitzpatrick, B. Gildea, K. H. Petersen, H. F. Hansen, T. Koch, M. Egholm, O. Buchardt, P. E. Nielsen, J. Coull, R. H. Berg, J. Pept. Sci., 1, 175 (1995); and
5) T. Koch, H. F. Hansen, P. Andersen, T. Larsen, H. G. Batz, K. Otteson, H. drum, J. Pept. Res., 49, 80 (1997).

The antisense oligomer described herein may be in the form of a pharmaceutically acceptable salt, in the form of a hydrate, or in the form of a hydrate of a pharmaceutically acceptable salt thereof.

Examples of the pharmaceutically acceptable salt of the antisense oligomer described herein include alkali metal salts such as a sodium salt, a potassium salt, and a lithium salt; alkaline earth metal salts such as a calcium salt, and a magnesium salt; metal salts such as an aluminum salt, an iron salt, a zinc salt, a copper salt, a nickel salt, and a cobalt salt; an ammonium salt; organic amine salts such as a t-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzyl-phenethylamine salt, a piperazine salt, a tetramethylammonium salt, and a tris(hydroxymethyl)aminomethane salt; hydrohalide salts such as a hydrofluoride salt, a hydrochloride salt, a hydrobromide salt, and a hydroiodide salt; inorganic acid salts such as a nitrate salt, a perchlorate salt, a sulfate salt, and a phosphate salt; lower alkanesulfonate salts such as a methanesulfonate salt, a trifluoromethanesulfonate salt, and an ethanesulfonate salt; arylsulfonate salts such as a benzenesulfonate salt, and p-toluenesulfonate salt; organic acid salts such as an acetate salt, a malate salt, a fumarate salt, a succinate salt, a citrate salt, a tartrate salt, an oxalate salt, and a maleate salt; and amino acid salts such as a glycine salt, a lysine salt, an arginine salt, an ornithine salt, a glutamate salt, an aspartate salt. These salts can be produced by a known method. Alternatively the antisense oligomer described herein may be in the form of a hydrate thereof.

The antisense oligomer described herein may have any of the groups represented in the following chemical formulae (1) to (3) at its 5'-end. Preferably, it is either group (1) or (2).

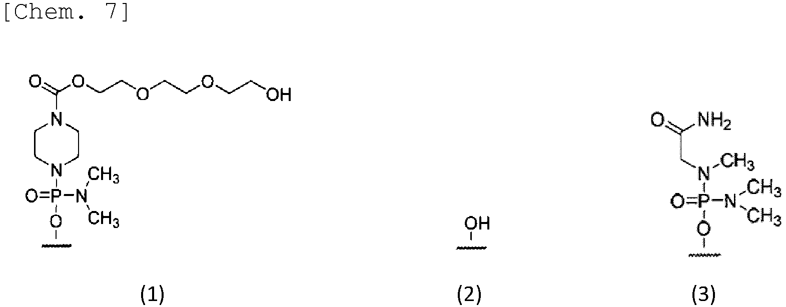

Hereinafter, the groups represented by (1), (2), and (3) above are referred to as "group (1)", "group (2)", and "group (3)", respectively.

The base sequence of the antisense oligomer described herein is not limited, and for example, the antisense oligomer may comprise four consecutive purine bases in its base sequence. Further, at least two of the four consecutive purine bases may be guanine.

In one embodiment, the antisense oligomer described herein comprises or consists of (i) a base sequence selected from the group consisting of SEQ ID NO: 1 to 10, (ii) a base sequence having a sequence identity of 80% or more, 85% or more, 86% or more, 87% or more, 88% or more, 89% or more, 90% or more, 910 or more, 92% or more, 93% or more, 94% or more, 95% or more, 96% or more, 97% or more, 98% or more, or 99% or more to the base sequence selected from the group consisting of SEQ ID NO: 1 to 10, or (iii) a basic sequence wherein one or several bases are added, deleted or substituted in the base sequence selected from the group consisting of SEQ ID NO: 1 to 10.

The term "identity" of the base sequences as used herein means a proportion of matched bases when two base sequences are aligned with each other. The sequence identity can be determined by using FASTA (Science 227 (4693): 1435-1441, (1985)) or the algorithm of Karlin and Altschul, BLAST (Basic Local Alignment Search Tool) (Proc. Natl. Acad. Sci. USA 872264-2268, 1990; Proc Natl Acad Sci USA 90: 5873, 1993). Programs based on the algorithm of BLAST and referred to as blastn, blastx, tblastn, and tblastx have been developed (Altschul SF, et al: J Mol Biol 215: 403, 1990). When blastn is used for base sequence analysis, parameters are set to, for example, score = 100, and wordlength = 12. When BLAST and Gapped BLAST programs are used, default parameters for each program are used.

As used herein, the term "several" in the base sequence wherein one or several bases are added, deleted, or substituted means 2, 3, 4, 5, 6, 7, 8, 9, or 10.

In one embodiment, the antisense oligomer described herein is one that targets a sequence ranging from positions 115937 to 115981 of a genome sequence (SEQ ID NO: 11) of the insertion mutant of the fukutin gene wherein an SVA retrotransposon sequence (GenBank ACCESSION: AB185332) is inserted into the genome sequence of the fukutin gene (GenBank ACCESSION: AB038490) or one that does not target the sequence of the above range. Examples of the antisense oligomer that targets a sequence ranging from positions 115937 to 115981 of SEQ ID NO: 11 include antisense oligomers comprising a base sequence selected from the group consisting of SEQ ID NO: 1 to 7. Examples of the antisense oligomer that does not targets a sequence ranging from positions 115937 to 115981 of SEQ ID NO: 11 include antisense oligomers comprising a base sequence selected from the group consisting of SEQ ID NO: 8 to 10.

In one embodiment, the antisense oligomer described herein is a conjugate to which a functional peptide, for example, cell-permeable peptide (CPP) is attached. Known functional peptides or commercially-available functional peptides can be used herein. Examples of the functional peptides that can be used herein include an arginine-rich peptide disclosed in International Publication No. 2008/036127; a peptide targeting organs disclosed in International Publication No. 2009/005793, for example, RXR, or RBR; and a peptide comprising amino acid subunits disclosed in International Publication No. 2012/150960. The cell-permeable peptide (CPP) can pass through cell membranes of mammalian cells, and accordingly, it represents a short peptide sequence having 10 to about 30 amino acids capable of improving cell drug delivery (see, e.g., Hum Mol Genet. 2011 Aug 15; 20(16): 3151-3160; Pharmacology & Therapeutics 154 (2015) 78-86). Known CPPs or commercially-available CPPs can be used herein. Examples of the CPPs that can be used herein include, the CPPs listed in Pharmacology & Therapeutics 154 (2015) 78-86, p.80, Table 1, such as TAT(48-60), penetratin, polyarginine, Oct4, WT1-pTj, DPV3, transportan, MAP, VP22, Rep1, KW, KFGF, FGF12, integrin β3 peptide, C105Y, TP2; and CPPs listed in Japanese Translation of PCT International Application Publication No. 2017-500856 (International Publication No. 2015/089487), paragraph [0085], Table 1, such as DPV10/6, DPV15b, YM-3, Tat, LR11, C45D18, Lyp-1, Lyp-2, BMV GAG, hLF1-22, C45D18, LR20. The CPPs are commercially available from Funakoshi, Co., Ltd., for example. Commercially available CPPs such as TAT (Funakoshi, Co., Ltd.), penetratin (Funakoshi, Co., Ltd.), or known CPPs such as R8 can be used herein. Examples of preferable CPPs that can be used herein include hLIMK, TAT, penetratin, and R8 (see, e.g., International Publication Nos. 2016/187425, 2018/118662, 2018/118599, and 2018/118627, and EBioMedicine 45 (2019) 630-645). The CPP can be directly bound to the antisense oligomer described herein, or can be bound via a linker capable of binding the CPP to the antisense oligomer. Known linkers can be used herein. Examples of the linker include those described in Japanese Translation of PCT International Application Publication No. 2017-500856 (International Publication No. 2015/089487), International Publication Nos. 2015/089487, 2009-073809, 2013/075035, 2015/105083, 2014/179620, 2015/006740, and 2017/010575. Examples of preferable linkers that can be used herein include 4-maleimidobutyrate, a linker capable of binding to the functional peptide or antisense oligomer described herein via disulfide bond. The conjugate as used herein can be prepared by a method known to those skilled in the art.

The pharmaceutical composition described herein may be formulated by appropriately blending a pharmaceutically acceptable carrier or additive (and optionally the sugar alcohol of the present invention). The pharmaceutically acceptable carrier or additive and formulation for the pharmaceutical composition is the same as those described for the nephrotoxicity reducing agent of the present invention except that the antisense nucleic acid is the active ingredient.

In one embodiment, the nephrotoxicity reducing agent of the present invention is used and/or added to the pharmaceutical composition in an amount such that the concentration of the sugar alcohol in the pharmaceutical composition described herein is 1 mg/mL to 400 mg/mL, for example, 2.5 mg/mL to 200 mg/mL. In one embodiment, the nephrotoxicity reducing agent of the present invention is used and/or added to the pharmaceutical composition in an amount such that the concentration of the sugar alcohol in the pharmaceutical composition is 1 mg/mL or more, 2.5 mg/mL or more, 3 mg/mL or more, 4 mg/mL or more, 5 mg/mL or more, 10 mg/mL or more, 15 mg/mL or more, 20 mg/mL or more, 30 mg/mL, or 40 mg/mL or more. Further, the nephrotoxicity reducing agent of the present invention is used and/or added to the pharmaceutical composition in an amount such that the concentration of the sugar alcohol in the pharmaceutical composition is 400 mg/mL or less, 350 mg/mL or less, 300 mg/mL or less, 250 mg/mL or less, or 200 mg/mL or less, for example.

In one embodiment, the concentration of the antisense oligomer in the pharmaceutical composition described herein is 0.5 mg/mL to 200 mg/mL, for example, 6 mg/mL to 200 mg/mL. In one embodiment, the concentration of the antisense oligomer in the pharmaceutical composition described herein may be 0.5 mg/mL or more, 1 mg/mL or more, 2 mg/mL or more, 3 mg/mL or more, 4 mg/mL or more, 5 mg/mL or more, or 6 mg/mL or more, and may be 200 mg/mL or less, 180 mg/mL or less, 150 mg/mL or less, 140 mg/mL or less, or 130 mg/mL or less.

In one embodiment, the present invention relates to a nephrotoxicity reducing agent for a pharmaceutical composition comprising an antisense oligomer, wherein the nephrotoxicity reducing agent comprises a sugar alcohol and is used in an amount such that the weight ratio of the sugar alcohol is 0.05 to 90, 0.05 to 30, for example, 0.1 to 13.3 per 1 of the antisense oligomer. The pharmaceutical composition comprising an antisense oligomer and the sugar alcohol are as described above. In one embodiment, the nephrotoxicity reducing agent of the present invention is used in an amount such that the weight ratio of the sugar alcohol is 0.05 or more, 0.1 or more, 0.15 or more, 0.2 or more, 0.3 or more, 0.4 or more, 0.5 or more, 0.6 or more, 0.7 or more, 0.8 or more, 0.9 or more, 1 or more, 2 or more, or 5 or more per 1 of the antisense oligomer. In one embodiment, the nephrotoxicity reducing agent of the present invention is used in an amount such that the weight ratio of the sugar alcohol is 90 or less, 80 or less, 70 or less, 60 or less, 50 or less, 40 or less, 30 or less, 25 or less, 20 or less, 15 or less, 13.3 or less, or 10 or less per 1 of the antisense oligomer.

In one embodiment, the nephrotoxicity reducing agent of the present invention is comprised in the pharmaceutical composition described herein and administered therewith. For example, the antisense oligomer described herein is optionally lyophilized with a carrier such as glucose, and then dissolved in a solvent such as water for injection to prepare a pharmaceutical composition. The resulting pharmaceutical composition may be mixed with the nephrotoxicity reducing agent described herein, and the amount of the mixture may then be optionally adjusted with a solvent to administer to a subject. Alternatively, for example, the antisense oligomer described herein is lyophilized with the nephrotoxicity reducing agent of the present invention, and then dissolved in a solvent such as water for injection to prepare a pharmaceutical composition. The amount thereof may then be optionally adjusted with a solvent to administer to a subject.

In another embodiment, the nephrotoxicity reducing agent of the present invention is not comprised in the pharmaceutical composition described herein, and is administered separately from (simultaneously or sequentially with) the pharmaceutical composition described herein. For example, a pharmaceutical composition obtained by dissolving a lyophilized agent of the antisense oligomer described herein in a solvent such as water for injection may be administered to a subject separately from the nephrotoxicity reducing agent described herein. As used herein, administering the nephrotoxicity reducing agent and the pharmaceutical composition "simultaneously" means administering the nephrotoxicity reducing agent and the pharmaceutical composition at the same time point. As used herein, administering the nephrotoxicity reducing agent and the pharmaceutical composition "sequentially" means administering each of them at a different time point. Specifically, the pharmaceutical composition can be administered before or after the nephrotoxicity reducing agent is administered, and in this case, a dosing interval between the nephrotoxicity reducing agent and the pharmaceutical composition is not limited, and may be, for example, several minutes, several hours, or up to about a day.

As used herein, examples of the subject to whom the pharmaceutical composition and/or nephrotoxicity reducing agent are administered include, but not limited to, mammals, such as primates such as humans, laboratory animals such as rats, mice, and Norway rats, and farm animals such as pigs, cattle, horses, and sheep, and preferred are humans.

The dose of the pharmaceutical composition and/or nephrotoxicity reducing agent when administered can be adjusted in consideration of the types of the antisense oligomer comprised in the pharmaceutical composition and the sugar alcohol comprised in the nephrotoxicity reducing agent, the dosage form of the pharmaceutical composition and nephrotoxicity reducing agent, the condition of the subject such as age or body weight, the administration route, and the nature and symptoms of the disease. The amount of the antisense oligomer can be in a range of 0.1 mg to 10 g/day/person, for example, in a range of 1 mg to 1 g/day/person, for example, or in a range of 10 mg to 100 mg/day/person, for example, and the amount of the sugar alcohol can be in a range of 0.1 mg to 200 g/day/person, for example, in a range of 1 mg to 100 g/day/person for example, in a range of 10 mg to 50 g/day/person, for example, in a range of 100 mg to 50 g/day/person, for example, in a range of 100 mg to 40 g/day/person, for example, in a range of 100 mg to 30 g/day/person, for example, in a range of 100 mg to 20 g/day/person, for example, in a range of 1 g to 20 g/day/person, for example, in a range of 2 g to 20 g/day/person, for example, in a range of 1 g to 10 g/day/person for example, or in a range of 100 mg to 1 g/day/person, for example. The number and frequency of administration are not limited, but for example, the administration can be performed once or two to three times a day at the interval of one day or two to three days. Further, for example, the administration can be performed only once, or another administration may be performed a few days later for a total of two administrations.

In one embodiment, the present invention relates to a method for reducing nephrotoxicity for a pharmaceutical composition comprising an antisense oligomer or a method for producing a pharmaceutical composition comprising an antisense oligomer with reduced nephrotoxicity, comprising adding a sugar alcohol in an amount such that the concentration of the sugar alcohol in the pharmaceutical composition is 1 mg/mL to 400 mg/mL. In the present embodiment, the antisense oligomer, the pharmaceutical composition, the sugar alcohol, the concentration of the sugar alcohol in the pharmaceutical composition and the like are as described herein.

In one embodiment, the present invention relates to a method for reducing nephrotoxicity of an antisense oligomer in a subject to whom the antisense oligomer or the pharmaceutical composition comprising the antisense oligomer has been administered, comprising administering a sugar alcohol or a nephrotoxicity reducing agent to the subject, wherein the sugar alcohol is administered in an amount such that the weight ratio of the sugar alcohol is 0.05 to 90 or 0.05 to 30 per 1 of the antisense oligomer. In the present embodiment, the antisense oligomer, the pharmaceutical composition, the sugar alcohol, the nephrotoxicity reducing agent, the weight ratio of the sugar alcohol per 1 of the antisense oligomer and the like are as described herein.

In one embodiment, the present invention relates to a pharmaceutical composition comprising an antisense oligomer with reduced nephrotoxicity, comprising a nephrotoxicity reducing agent at the concentration of 1 mg/mL to 400 mg/mL, the nephrotoxicity reducing agent comprising a sugar alcohol. In the present embodiment, the antisense oligomer, the pharmaceutical composition, the sugar alcohol, the concentration of the sugar alcohol in the pharmaceutical composition and the like are as described herein.

### Examples

### [Example 1: Production of Antisense Oligomer (PMO)]

The antisense oligomers (PMO Nos. 1 to 10 (SEQ ID NO: 1 to 10)) listed in Table 1 were synthesized according to the method described in International Publication No. WO2013/100190. The theoretical value and measured value measured by ESI-TOF-MS of the molecular weight of each antisense oligomer are also shown.

### [Table 1]

**Table 1: Synthesized antisense oligomers (PMO No. 1 to 10)**

| PMO No. | Sequence | 5'-end | Molecular weight | | SEQ ID NO. |
|---|---|---|---|---|---|
| | | | Theoretical value | Measured value | |
| 1 | GCTCGGCATCAGAGGGAGACCG | group (2) | 7353.55 | 7353.93 | 1 |
| 2 | CTCGGCATCAGAGGGAGACC | group (2) | 6643.31 | 6643.91 | 2 |
| 3 | CGGCATCAGAGGGAGAC | group (2) | 5682.99 | 5682.27 | 3 |
| 4 | CCTTGGCTCGGCATCAGAGG | group (2) | 6625.29 | 6625.31 | 4 |
| 5 | CATCAGAGGGAGACCGTGTAA | group (2) | 7021.45 | 7021.88 | 5 |
| 6 | CTCGTGCATCAGAGGGAGACC | group (2) | 6973.42 | 6974.13 | 6 |
| 7 | CTCGCATCAGAGGGAGACC | group (2) | 6288.19 | 6287.87 | 7 |
| 8 | AGGAGGGCAGCGAACTCCTGGT TGT | group (2) | 8383.90 | 8383.85 | 8 |
| 9 | GTGGTTGAGCCAGAAGCTGGGA ATT | group (2) | 8422.91 | 8422.85 | 9 |
| 10 | GTTGCCTCCGGTTCTGAAGGTGT TC | group (1) | 8643.00 | 8642.94 | 10 |

"Group (1)" and "group (2)" in the table above are as follows.

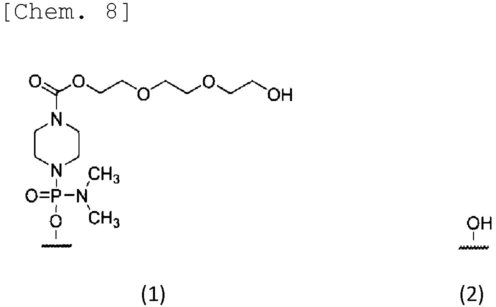

### [Example 2: Evaluation of Nephrotoxicity Reducing Effects of D-Mannitol]

Safety evaluation in the kidney was conducted on the antisense oligomers of PMO Nos. 1, 2, 3, 4, 6, 7, and 10 (SEQ ID NO: 1, 2, 3, 4, 6, 7, and 10) among those listed in Table 1 to verify their utility for pharmaceutical applications.

### (1) Evaluation Method

The antisense oligomers of PMO Nos. 1, 2, 6, 7, and 10 were each dissolved in water for injection comprising 0.9% sodium chloride (manufactured by Otsuka Pharmaceutical Co., Ltd.) to prepare a control dosing solution comprising each of the antisense oligomers at the concentrations shown in Tables 2, 3, 6, 7, and 8 and comprising no D-mannitol. Also, the antisense oligomers of PMO Nos. 3 and 4 were each dissolved in 5% glucose (manufactured by Otsuka Pharmaceutical Co., Ltd.) to prepare a control dosing solution comprising each of the antisense oligomers at the concentrations shown in Tables 4 and 5 and comprising no D-mannitol.

Next, the antisense oligomers of PMO Nos. 1, 2, 6, 7, and 10 were each dissolved in water for injection comprising 0.9% sodium chloride, and a solution obtained by diluting 20% D-mannitol (manufactured by Yoshindo, Inc.) with physiological saline (manufactured by Otsuka Pharmaceutical Co., Ltd.) or water for injection comprising 0.9% sodium chloride was added thereto to prepare a dosing solution comprising each of the antisense oligomers and D-mannitol at the concentrations shown in Tables 2, 3, 6, 7, and 8. Also, the antisense oligomers of PMO Nos. 3 and 4 (SEQ ID NO: 3 and 4) were each dissolved in a solution obtained by diluting 15% D-mannitol (manufactured by Terumo Corporation) with water for injection to prepare a dosing solution comprising each of the antisense oligomers and D-mannitol at the concentrations shown in Tables 4 and 5.

The obtained dosing solutions were administered into the tail vein of 6-week-old C57BL/6J male mice at 4, 10, and 20 mL/kg. Next day, serum was collected from the mice, and the blood urea nitrogen (BUN) values and blood creatinine (Cre) values were measured with the urease-GIDH method and the enzymatic method, respectively, using automatic biochemical analyzers JCA-BM6050 or JCA-BM8060 (manufactured by JEOL Ltd.). It was determined that the nephrotoxicity was reduced in a case where both of the BUN values and Cre values decreased in the mice to which the dosing solution comprising D-mannitol has been administered, compared to the mice to which the control dosing solution comprising no D-mannitol has been administered.

### (2) Evaluation Results

Since each of the antisense oligomers tested was confirmed that both of the BUN values and Cre values tended to decrease in the mice to which the dosing solution comprising D-mannitol has been administered, D-mannitol was confirmed to reduce nephrotoxicity. The results are shown in Tables 2, 3, 4, 5, 6, 7, and 8.

**[Table 2]**

| Table 2: BUN values and Cre values when administering antisense oligomer (PMO No. 1) (N=3) | | | | | | | |
|---|---|---|---|---|---|---|---|
| Amount of dosing solution (mL/kg) | Concentration of PMO (mg/mL) | Concentration of D-mannitol (mg/mL) | Weight ratio of D-mannitol/PMO | BUN (mg/dL) | Standard deviation of BUN (mg/dL) | Cre (mg/dL) | Standard deviation of Cre (mg/dL) |
| - | - | - | - | 33.5 | 0.5 | 0.14 | 0.04 |
| 20 | 15 | 0 | 0 | 129.4 | 36.6 | 0.32 | 0.10 |
| 20 | 15 | 2.5 | 0.167 | 75.5 | 27.4 | 0.36 | 0.09 |
| 20 | 15 | 10 | 0.667 | 34.2 | 5.6 | 0.13 | 0.04 |
| 20 | 15 | 25 | 1.667 | 32.4 | 1.6 | 0.10 | 0.02 |

| | | | | | | | |
|---|---|---|---|---|---|---|---|
| -: No administration | | | | | | | |

**[Table 3]**

| Table 3: BUN values and Cre values when administering antisense oligomer (PMO No. 2) (N=3) | | | | | | | |
|---|---|---|---|---|---|---|---|
| Amount of dosing solution (mL/kg) | Concentration of PMO (mg/mL) | Concentration of D-mannitol (mg/mL) | Weight ratio of D-mannitol/PMO | BUN (mg/dL) | Standard deviation of BUN (mg/dL) | Cre (mg/dL) | Standard deviation of Cre (mg/dL) |
| - | - | - | - | 44.6 | 10.3 | 0.13 | 0.01 |
| 10 | 30 | 0 | 0 | 166.2 | 79.4 | 0.56 | 0.41 |
| 10 | 30 | 5 | 0.167 | 76.4 | 51.9 | 0.23 | 0.19 |
| 10 | 30 | 20 | 0.667 | 37.2 | 5.3 | 0.13 | 0.04 |
| 10 | 30 | 50 | 1.667 | 39.5 | 4.6 | 0.10 | 0.01 |

| | | | | | | | |
|---|---|---|---|---|---|---|---|
| -: No administration | | | | | | | |

**[Table 4]**

| Table 4: BUN values and Cre values when administering antisense oligomer (PMO No. 3) (N=3) | | | | | | | |
|---|---|---|---|---|---|---|---|
| Amount of dosing solution (mL/kg) | Concentration of PMO (mg/mL) | Concentration of D-mannitol (mg/mL) | Weight ratio of D-mannitol/PMO | BUN (mg/dL) | Standard deviation of BUN (mg/dL) | Cre (mg/dL) | Standard deviation of Cre (mg/dL) |
| - | - | - | - | 25.4 | 1.0 | 0.07 | 0.01 |
| 10 | 6 | 0 | 0 | 240.0 | 5.6 | 2.22 | 0.04 |
| 10 | 6 | 50 | 8.333 | 28.7 | 1.1 | 0.06 | 0.01 |
| 10 | 20 | 0 | 0 | 259.2 | 22.4 | 1.82 | 0.14 |
| 10 | 20 | 50 | 2.5 | 28.1 | 5.4 | 0.07 | 0.01 |

| | | | | | | | |
|---|---|---|---|---|---|---|---|
| -: No administration | | | | | | | |

**[Table 5]**

| Table 5: BUN values and Cre values when administering antisense oligomer (PMO No. 4) | | | | | | | |
|---|---|---|---|---|---|---|---|
| (N=5) | | | | | | | |
| Amount of dosing solution (mL/kg) | Concentration of PMO (mg/mL) | Concentration of D-mannitol (mg/mL) | Weight ratio of D-mannitol/PMO | BUN (mg/dL) | Standard deviation of BUN (mg/dL) | Cre (mg/dL) | Standard deviation of Cre (mg/dL) |
| - | - | - | - | 31.7 | 2.8 | 0.08 | 0.01 |
| 4 | 75 | 0 | 0 | 159.2 | 87.0 | 1.12 | 0.85 |
| 4 | 75 | 50 | 0.667 | 41.4 | 3.5 | 0.09 | 0.01 |

| | | | | | | | |
|---|---|---|---|---|---|---|---|
| -: No administration | | | | | | | |

**[Table 6]**

| Table 6: BUN values and Cre values when administering antisense oligomer (PMO No. 6) (N=3) | | | | | | | |
|---|---|---|---|---|---|---|---|
| Amount of dosing solution (mL/kg) | Concentration of PMO (mg/mL) | Concentration of D-mannitol (mg/mL) | Weight ratio of D-mannitol/PMO | BUN (mg/dL) | Standard deviation of BUN (mg/dL) | Cre (mg/dL) | Standard deviation of Cre (mg/dL) |
| - | - | - | - | 26.6 | 2.6 | 0.11 | 0.01 |
| 10 | 100 | 0 | 0 | 128.8 | 70.6 | 0.41 | 0.35 |
| 10 | 100 | 20 | 0.2 | 27.6 | 2.1 | 0.11 | 0.02 |
| 10 | 100 | 50 | 0.5 | 35.1 | 5.9 | 0.09 | 0.04 |

| | | | | | | | |
|---|---|---|---|---|---|---|---|
| -: No administration | | | | | | | |

**[Table 7]**

| Table 7: BUN values and Cre values when administering antisense oligomer (PMO No. 7) (N=3) | | | | | | | |
|---|---|---|---|---|---|---|---|
| Amount of dosing solution (mL/kg) | Concentration of PMO (mg/mL) | Concentration of D-mannitol (mg/mL) | Weight ratio of D-mannitol/PMO | BUN (mg/dL) | Standard deviation of BUN (mg/dL) | Cre (mg/dL) | Standard deviation of Cre (mg/dL) |
| - | - | - | - | 31.9 | 5.0 | 0.18 | 0.05 |
| 20 | 100 | 0 | 0 | 238.4 | 31.7 | 1.43 | 0.12 |
| 20 | 100 | 10 | 0.1 | 30.1 | 1.8 | 0.07 | 0.01 |
| 20 | 100 | 50 | 0.5 | 30.8 | 1.5 | 0.08 | 0.01 |
| 20 | 100 | 200 | 2 | 32.0 | 2.6 | 0.10 | 0.01 |

| | | | | | | | |
|---|---|---|---|---|---|---|---|
| -: No administration | | | | | | | |

**[Table 8]**

| Table 8: BUN values and Cre values when administering antisense oligomer (PMO No. 10) (N=3) | | | | | | | |
|---|---|---|---|---|---|---|---|
| Amount of dosing solution (mL/kg) | Concentration of PMO (mg/mL) | Concentration of D-mannitol (mg/mL) | Weight ratio of D-mannitol/PMO | BUN (mg/dL) | Standard deviation of BUN (mg/dL) | Cre (mg/dL) | Standard deviation of Cre (mg/dL) |
| 20* | 0 | 0 | - | 32.4 | 3.4 | 0.09 | 0.01 |
| 20 | 100 | 0 | 0 | 151.4 | 46.9 | 0.40 | 0.19 |
| 20 | 100 | 200 | 2 | 33.9 | 2.1 | 0.08 | 0.01 |

| | | | | | | | |
|---|---|---|---|---|---|---|---|
| *: Administered with Dulbecco's PBS(-) | | | | | | | |

The above results showed that D-mannitol reduces the nephrotoxicity due to the administration of antisense oligomers.

### [Example 3: Evaluation of Nephrotoxicity Reducing Effects of D-Sorbitol]

Safety evaluation in the kidney was conducted on the antisense oligomers of PMO Nos. 2 and 3 (SEQ ID NO: 2 and 3) among those listed in Table 1 to verify their utility for pharmaceutical applications.

### (1) Evaluation Method

The antisense oligomer of PMO No. 2 was dissolved in physiological saline to prepare a control dosing solution comprising the antisense oligomer at the concentration shown in Table 9 and comprising no D-sorbitol. Also, the antisense oligomer of PMO No. 3 was dissolved in Dulbecco's PBS(-) (manufactured by Nissui Pharmaceutical Co., Ltd.) to prepare a control dosing solution comprising the antisense oligomer at the concentration shown in Table 10 and comprising no D-sorbitol.

Next, the antisense oligomer of PMO No. 2 was dissolved in a solution obtained by dissolving D-sorbitol (manufactured by Maruishi Pharmaceutical Co., Ltd.) with physiological saline to prepare a dosing solution comprising the antisense oligomer and D-sorbitol at the concentrations shown in Table 9. Also, the antisense oligomer of PMO No. 3 was dissolved in a solution obtained by dissolving D-sorbitol with water for injection to prepare a dosing solution comprising the antisense oligomer and D-sorbitol at the concentrations shown in Table 10.

The obtained dosing solutions were administered into the tail vein of 6-week-old C57BL/6J male mice (N=3) at 4 and 10 mL/kg. Next day, serum was collected from the mice, and the blood urea nitrogen (BUN) values and blood creatinine (Cre) values were measured with the urease-GIDH method and the enzymatic method, respectively, using automatic biochemical analyzers JCA-BM8060 (manufactured by JEOL Ltd.). It was determined that the nephrotoxicity was reduced in a case where both of the BUN values and Cre values decreased in the mice to which the dosing solution comprising D-sorbitol has been administered, compared to the mice to which the dosing solution comprising no D-sorbitol has been administered.

### (2) Evaluation Results

Since each of the antisense oligomers tested was confirmed that both of the BUN values and Cre values tended to decrease in the mice to which the dosing solution comprising D-sorbitol has been administered, D-sorbitol was confirmed to reduce nephrotoxicity. The results are shown in Tables 9 and 10.

**[Table 9]**

| Table 9: BUN values and Cre values when administering antisense oligomer (PMO No. 2) | | | | | | | |
|---|---|---|---|---|---|---|---|
| Amount of dosing solution (mL/kg) | Concentration of PMO (mg/mL) | Concentration of D-sorbitol (mg/mL) | Weight ratio of D-sorbitol/PMO | BUN (mg/dL) | Standard deviation of BUN (mg/dL) | Cre (mg/dL) | Standard deviation of Cre (mg/dL) |
| - | - | - | - | 32.4 | 5.6 | 0.08 | 0.02 |
| 4 | 50 | 0 | 0 | 152.5 | 75.7 | 1.21 | 0.97 |
| 4 | 50 | 6.25 | 0.125 | 154.6 | 20.5 | 0.57 | 0.24 |
| 4 | 50 | 12.5 | 0.25 | 136.7 | 27.5 | 0.54 | 0.20 |
| 4 | 50 | 25 | 0.5 | 56.4 | 41.2 | 0.15 | 0.15 |
| 4 | 50 | 50 | 1 | 39.4 | 2.4 | 0.05 | 0.02 |

| | | | | | | | |
|---|---|---|---|---|---|---|---|
| -: No administration | | | | | | | |

**[Table 10]**

| Table 10: BUN values and Cre values when administering antisense oligomers (PMO No. 3) | | | | | | | |
|---|---|---|---|---|---|---|---|
| Amount of dosing solution (mL/kg) | Concentration of PMO (mg/mL) | Concentration of D-sorbitol (mg/mL) | Weight ratio of D-sorbitol/PMO | BUN (mg/dL) | Standard deviation of BUN (mg/dL) | Cre (mg/dL) | Standard deviation of Cre (mg/dL) |
| - | - | - | - | 38.0 | 1.7 | 0.08 | 0.00 |
| 10 | 6 | 0 | 0 | 236.2 | 78.7 | 1.34 | 0.66 |
| 10 | 6 | 50 | 8.333 | 39.9 | 3.6 | 0.08 | 0.02 |

| | | | | | | | |
|---|---|---|---|---|---|---|---|
| -: No administration | | | | | | | |

### [Example 4: Evaluation of Precipitation Suppressing Effects with D-Mannitol]

### Test Method

The amount of precipitation in the presence of ions assumed to be in urine and precipitation suppressing effects of D-mannitol were evaluated on the antisense oligomers of PMO Nos. 1, 2, 3, 5, 6, and 7 (SEQ ID NO: 1, 2, 3, 5, 6, and 7) among those listed in Table 1 to further verify their utility for pharmaceutical applications.

Each of the antisense oligomers was dissolved in physiological saline or an aqueous solution of D-mannitol, and the mixture was then mixed with an aqueous solution comprising potassium chloride and sodium chloride assumed to be in urine. Evaluation conditions (solution compositions) are shown in Tables 11 to 16. The absorbance at 620 nm of the solution thus mixed was measured using a plate reader, Infinite F200 Pro (manufactured by Tecan Group Ltd.) under heating conditions of 37°C. Assuming the precipitation of antisense oligomers in urine, the concentrations of the antisense oligomers, potassium chloride and sodium chloride vary depending on the amount of the antisense oligomer administered and/or the amount of urine. Accordingly, conditions were set under which the precipitation was observed using water for injection as a medium for each sequence. Another measurement was performed under the same conditions as obtained except that only the medium was changed to an aqueous solution of D-mannitol, and then the absorbance obtained under both the conditions was compared to each other. When the absorbance was increased, the precipitation was determined to be observed, and a time until the precipitation was observed and the increased value of the absorbance were evaluated. It was determined that the precipitation suppressing effect was presented when the aqueous solution of D-mannitol was used as the medium in a case where the time until the precipitation was observed was longer or in a case where the increased value of the absorbance was smaller than the case of using the water for injection as the medium.

**[Table 11]**

| Table 11: Evaluation conditions of precipitation suppression with D-mannitol on PMO No. 1 | | | | |
|---|---|---|---|---|
| PMO No. | PMO (mg/mL) | KC1 (mmol/mL) | NaCl (mmol/mL) | D-mannitol (mg/mL) |
| 1 | 24.75 | 100 | 77 | 0 |
| 1 | 24.75 | 100 | 77 | 135 |

**[Table 12]**

| Table 12: Evaluation conditions of precipitation suppression with D-mannitol on PMO No. 2 | | | | |
|---|---|---|---|---|
| PMO No. | PMO (mg/mL) | KCl (mmol/mL) | NaCl (mmol/mL) | D-mannitol (mg/mL) |
| 2 | 49.5 | 100 | 77 | 0 |
| 2 | 49.5 | 100 | 77 | 135 |

**[Table 13]**

| Table 13: Evaluation conditions of precipitation suppression with D-mannitol on PMO No. 3 | | | | |
|---|---|---|---|---|
| PMO No. | PMO (mg/mL) | KCl (mmol/mL) | NaCl (mmol/mL) | D-mannitol (mg/mL) |
| 3 | 9.9 | 100 | 77 | 0 |
| 3 | 9.9 | 100 | 77 | 135 |

**[Table 14]**

| Table 14: Evaluation conditions of precipitation suppression with D-mannitol on PMO No. 5 | | | | |
|---|---|---|---|---|
| PMO No. | PMO (mg/mL) | KCl (mmol/mL) | NaCl (mmol/mL) | D-mannitol (mg/mL) |
| 5 | 49.5 | 100 | 77 | 0 |
| 5 | 49.5 | 100 | 77 | 135 |

**[Table 15]**

| Table 15: Evaluation conditions of precipitation suppression with D-mannitol on PMO No. 6 | | | | |
|---|---|---|---|---|
| PMO No. | PMO (mg/mL) | KCl (mmol/mL) | NaCl (mmol/mL) | D-mannitol (mg/mL) |
| 6 | 49.5 | 100 | 77 | 0 |
| 6 | 49.5 | 100 | 77 | 135 |

**[Table 16]**

| Table 16: Evaluation conditions of precipitation suppression with D-mannitol on PMO No. 7 | | | | |
|---|---|---|---|---|
| PMO No. | PMO (mg/mL) | KCl (mmol/mL) | NaCl (mmol/mL) | D-mannitol (mg/mL) |
| 7 | 264 | 400 | 154 | 0 |
| 7 | 264 | 400 | 154 | 120 |

### Test Results

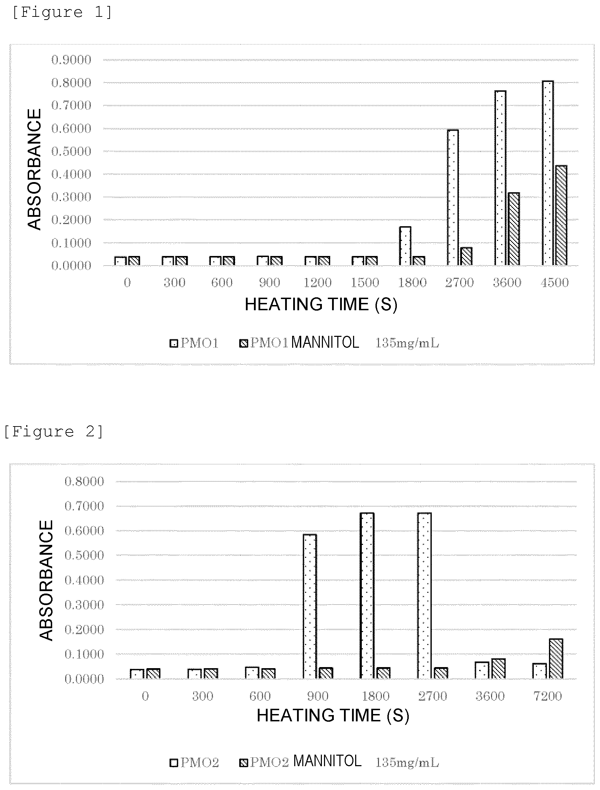

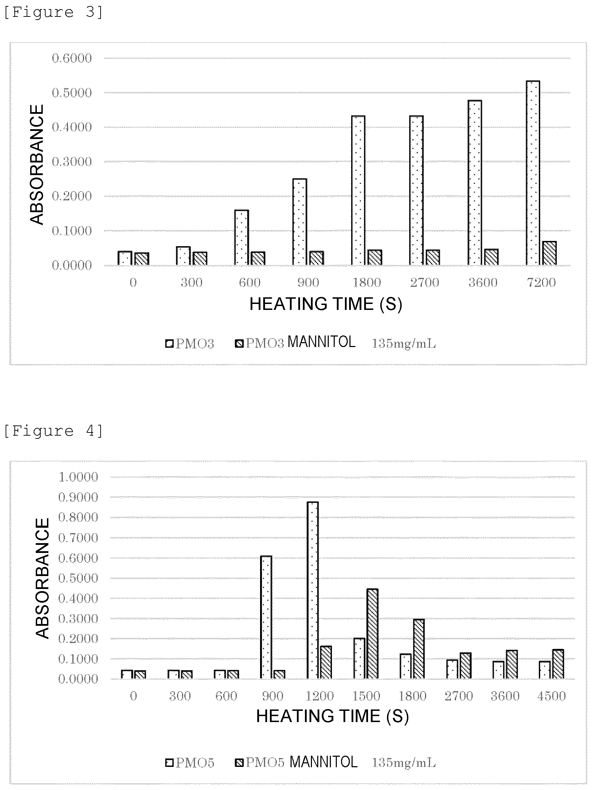

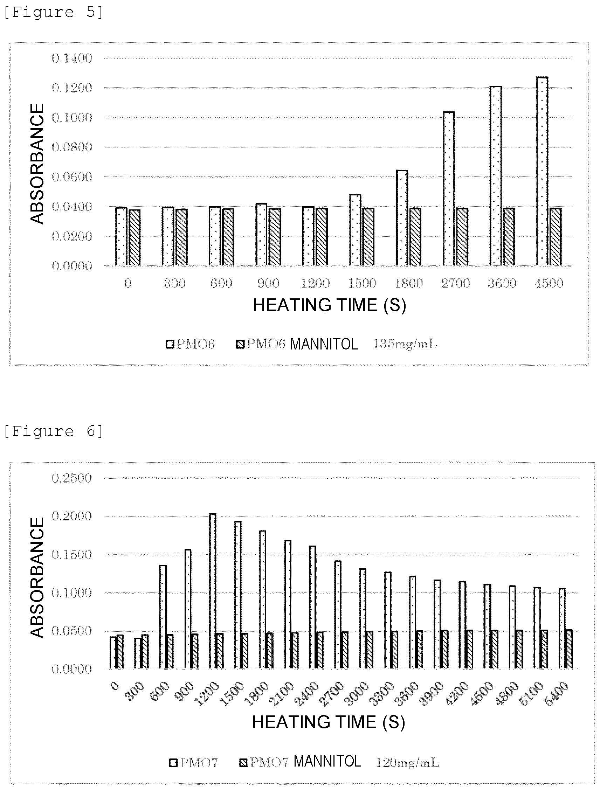

Since each of the antisense oligomers tested prolonged the time until the precipitation was observed and suppressed the increase of the absorbance in all the case of using the aqueous solution of D-mannitol as the medium, D-mannitol was indicated to suppress the precipitation of antisense oligomers. The respective results are shown in Figures 1 to 6.

The above results showed that D-mannitol suppresses the precipitation of the antisense oligomers in urine.

## Claims

1. A nephrotoxicity reducing agent for a pharmaceutical composition comprising an antisense oligomer, wherein the nephrotoxicity reducing agent comprises a sugar alcohol and is used in an amount such that a concentration of the sugar alcohol in the pharmaceutical composition is 1 mg/mL to 400 mg/mL.

2. The nephrotoxicity reducing agent according to claim 1, wherein the nephrotoxicity reducing agent is used in an amount such that the concentration of the sugar alcohol in the pharmaceutical composition is 2.5 mg/mL to 200 mg/mL.

3. The nephrotoxicity reducing agent according to claim 1 or 2, wherein a concentration of the antisense oligomer in the pharmaceutical composition is 0.5 mg/mL to 200 mg/mL.

4. A nephrotoxicity reducing agent for a pharmaceutical composition comprising an antisense oligomer, wherein the nephrotoxicity reducing agent comprises a sugar alcohol and is used in an amount such that a weight ratio of the sugar alcohol is 0.05 to 30 per 1 of the antisense oligomer.

5. The nephrotoxicity reducing agent according to claim 4, wherein the nephrotoxicity reducing agent is used in an amount such that the weight ratio of the sugar alcohol is 0.1 to 13.3 per 1 of the antisense oligomer.

6. The nephrotoxicity reducing agent according to any one of claims 1 to 5, wherein the sugar alcohol is selected from the group consisting of mannitol, sorbitol, and a combination thereof.

7. The nephrotoxicity reducing agent according to any one of claims 1 to 6, wherein the antisense oligomer is a morpholino oligomer.

8. The nephrotoxicity reducing agent according to any one of claims 1 to 7, wherein the antisense oligomer is a phosphorodiamidate morpholino oligomer.

9. The nephrotoxicity reducing agent according to any one of claims 1 to 8, wherein the 5'-end of the antisense oligomer is any group represented by the following chemical formulae (1) and (2):

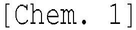

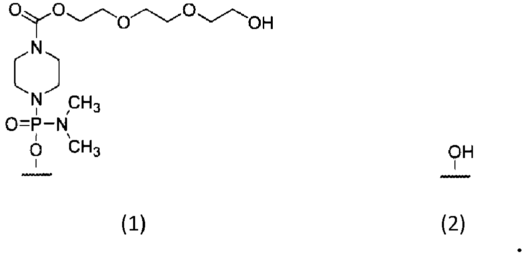

10. The nephrotoxicity reducing agent according to any one of claims 1 to 9, wherein the antisense oligomer comprises four consecutive purine bases in its base sequence.

11. The nephrotoxicity reducing agent according to claim 10, wherein at least two of the four consecutive purine bases are guanine.

12. The nephrotoxicity reducing agent according to any one of claims 1 to 11, wherein the antisense oligomer comprises a base sequence selected from the group consisting of SEQ ID NOs: 1 to 10.

13. The nephrotoxicity reducing agent according to any one of claims 1 to 12, wherein the antisense oligomer targets a sequence ranging from positions 115937 to 115981 of a base sequence set forth as SEQ ID NO: 11.

14. The nephrotoxicity reducing agent according to any one of claims 1 to 12, wherein the antisense oligomer does not target a sequence ranging from positions 115937 to 115981 of a base sequence set forth as SEQ ID NO: 11.

15. A method for reducing nephrotoxicity for a pharmaceutical composition comprising an antisense oligomer, comprising adding a sugar alcohol in an amount such that a concentration of the sugar alcohol in the pharmaceutical composition is 1 mg/mL to 400 mg/mL.

16. A method for reducing nephrotoxicity of an antisense oligomer in a subject to whom the antisense oligomer has been administered, comprising administering a sugar alcohol to the subject, wherein the sugar alcohol is administered in an amount such that a weight ratio of the sugar alcohol is 0.05 to 30 per 1 of the antisense oligomer.

17. A pharmaceutical composition comprising an antisense oligomer with reduced nephrotoxicity, wherein the pharmaceutical composition comprises a nephrotoxicity reducing agent comprising a sugar alcohol at a concentration of 1 mg/mL to 400 mg/mL.
